# EUROPÄISCHE PATENTSCHRIFT

(11) **EP 0 826 372 B1**
(45) Veröffentlichungstag und Bekanntmachung des Hinweises auf die Patenterteilung: **24.03.2004**
(21) Anmeldenummer: 97115225.1
(22) Anmeldetag: 03.09.1997
(51) Int. Cl.: A61K 35/78

(54) **Verfahren zur Herstellung eines stabilen, homogenen, von Folgeprodukten freien oder nahezu freien Extraktes**
Process for the preparation of a stable, homogeneous, from secondary reaction products free or nearly free extract
Procédé de préparation d'un extrait stable, homogène, exempt ou presque exempt de produits de réaction secondaires

(30) Priorität: 03.09.1996 CH 216696
(43) Veröffentlichungstag der Anmeldung: 04.03.1998
(73) Patentinhaber: Frutarom Schweiz AG, 8820 Wädenswil (CH)
(72) Erfinder: Kreuter, Matthias-Heinrich, Dr., 8880 Walenstadt (CH); Steiner, Rudolf, 8806 Bäch (CH)
(74) Vertreter: Zink-Wild, Markus Peter

(56) Entgegenhaltungen:
- EP-A- 0 347 493
- DATABASE WPI Section Ch, Week 8551 Derwent Publications Ltd., London, GB; Class A96, AN 85-319798 XP002021463 & JP 60 222 412 A (KOKANDO KK) , 7.November 1985
- DATABASE WPI Section Ch, Week 9208 Derwent Publications Ltd., London, GB; Class B04, AN 92-060692 XP002021464 & JP 04 005 237 A (NONOKAWA SHOJI YG) , 9.Januar 1992
- DATABASE WPI Section Ch, Week 9139 Derwent Publications Ltd., London, GB; Class B04, AN 91-286062 XP002021465 & JP 03 190 809 A (SHISEIDO KK) , 20.August 1991
- DATABASE WPI Section Ch, Week 9128 Derwent Publications Ltd., London, GB; Class D13, AN 91-204666 XP002021466 & JP 03 130 051 A (KOTOBUKI ACADEMY KK) , 3.Juni 1991
- DATABASE WPI Section Ch, Week 7711 Derwent Publications Ltd., London, GB; Class A14, AN 77-19650Y XP002021467 & SU 523 115 A (MOSC MAGARACH WINE) , 7.September 1976

## Beschreibung

Die vorliegende Erfindung betrifft ein Verfahren zur Herstellung eines stabilen, homogenen, von Folgeprodukten freien oder nahezu freien Extraktes, welcher das jeweils gewünschte genuine Substanzgemisch in vollständiger oder nahezu vollständiger Form enthält, wobei dieser Extrakt aus Pflanzen oder Teilen davon erhalten wird, welche im frischen und/oder getrockneten Zustand in bearbeiteter oder unbearbeiteter Form eingesetzt werden können.

Diese Erfindung betrifft auch ein Mittel zur Durchführung dieses Verfahrens.

Diese Erfindung betrifft auch einen stabilen, homogenen, von Folgeprodukten freien oder nahezu freien Extrakt aus Pflanzen oder Teilen davon.

Ueblicherweise werden Pflanzenextrakte durch Extraktion mit Alkoholen, Mischungen aus Wasser und Alkoholen oder auch nur mit Wasser hergestellt.

Wenn ein solcher Pflanzenextrakt in eine dickflüssige Form, ein sogenannter Spissumextrakt, oder in eine trockene Form, ein sogenannter Siccumextrakt, überführt werden soll, dann muss das vorhandene Lösungsmittel oder Lösungsmittelgemisch teilweise oder vollständig entfernt werden.

Dies geschieht üblicherweise unter Verdampfung des(der) Lösungsmittel(s) unter reduziertem Druck und bei erhöhten Temperaturen.

Diese Verfahren sind in Arzneibüchern, beispielsweise im Deutschen Arzneibuch (DAB) oder im Europäischen Arzneibuch (EuAB), beschrieben.

Wenn man beispielsweise Chelidonium majus L. nach einem solchen Verfahren extrahiert und anschliessend einengt und trocknet, dann werden Zersetzungs- und Polymerisationsprozesse sowie eine Sedimentation und eine Flotation der Inhaltsstoffe beobachtet.

Als Folge davon stellt das resultierende Produkt keine der eingesetzten Pflanzenmenge äquivalente Zubereitung dar, weil das so erhaltene Substanzgemisch nicht dem aus der Pflanze herausgelösten genuinen Substanzgemisch entspricht.

Wenn man beispielsweise Melilotus officinalis L. Lam. em.Thuill. nach einem oben genannten Verfahren extrahiert und anschliessend einengt und trocknet, dann stellt man einen Verlust an flüchtigen Komponenten, wie etwa Cumarine, fest.

Als Folge davon stellt auch dieses resultierende Produkt keine der eingesetzten Pflanzenmenge äquivalente Zubereitung dar, weil das so erhaltene Substanzgemisch nicht dem aus der Pflanze herausgelösten genuinen Substanzgemisch entspricht.

Wenn man beispielsweise Hypericum perforatum L. nach einem oben genannten Verfahren extrahiert, was normalerweise in einem Verhältnis von Pflanze zu eingesetztem Lösungsmittel von 1:1 bis 1:20, insbesondere 1:10, geschieht, dann werden die schwerlöslichen Anteile, wie etwa die Dianthrone, unvollständig aus der Pflanze extrahiert.

Als Folge davon stellt auch dieses resultierende Produkt keine der eingesetzten Pflanzenmenge äquivalente Zubereitung dar, weil das so erhaltene Substanzgemisch nicht dem aus der Pflanze herauslösbaren genuinen Substanzgemisch entspricht.

Wenn man beispielsweise Allium sativum L. nach einem oben genannten Verfahren extrahiert und anschliessend einengt und trocknet, dann werden während des gesamten Verfahrens chemische und/oder enzymatische Reaktionen zwischen einer oder mehrerer im Extrakt enthaltenen Substanz(en) festgestellt.

Diese Reaktionen werden mit dem in EP 0 347 493 beschriebenen Verfahren nahezu vollständig verhindert. Das gemäss diesem Verfahren erhaltene Produkt weist bei erhöhter Temperatur, d.h. bei einer Temperatur von 25°C und mehr, eine ungenügende Stabilität der Thiosulfinate, beispielsweise Alliin, auf.

Solche gemäss EP 0 347 493 erhaltene Produkte, welche über eine längere Zeitspanne, beispielsweise 1 Monat, bei einer Temperatur von mehr als 25°C gelagert worden sind, stellen somit nicht mehr eine dem aus der Pflanze herausgelösten genuinen Substanzgemisch äquivalente Zubereitung dar.

Wenn man einen Gerbstoffe und/oder Isoprenoide enthaltenden Pflanzenextrakt in eine verkapselbare Masse gemäss EP 0 496 705 überführt, dann werden bei der Verwendung von Polyethylenglykol-Sequenzen enthaltenden Trägermaterialien, wie etwa Polyethylenglykole, Polysorbate, während oder nach der Herstellung solcher Massen Sedimentationen, Flotationen, Inhomogenitäten und chemische Reaktionen festgestellt.

Es ist festgestellt worden, dass Pflanzenextrakte, welche Gerbstoffe und/oder Isoprenoide enthalten, bei der Einkapselung in eine Gelatinekapsel mit der Gelatinehülle reagieren und somit die Elastizität der Kapselhülle und deren Auflösungsverhalten im Magen oder im Darm negativ beeinflussen können.

Diesen Nachteil haben die in EP 0 464 274 A1 und EP 0 496 705 beschriebenen Produkte.

Dieses Verhalten von Gerbstoffen wird zum Beispiel bei der Herstellung von Leder ausgenutzt und ist seit hunderten von Jahren bekannt.

Dieses Verhalten von bestimmten Isoprenoiden, vor allem der Aldehydoderivate der Monoterpene und Sesquiterpene, insbesondere ihre Reaktivität mit Aminogruppen enthaltenden Verbindungen, ist auch allgemein bekannt und wird zur Herstellung von derivatisierten Weichgelatinekapseln mit verzögertem Auflösungsverhalten gezielt eingesetzt; siehe DAB 10, Kommentar K 20.

Gemäss DE PS 44 34 170 besteht die Aufgabe darin, peroral applizierbare Extrakte von Hypericum perforatum L. zur Verfügung zu stellen, welche eine gegenüber herkömmlichen Präparaten höhere Freisetzungsrate, also eine höhere Bioverfügbarkeit, der wirksamen Bestandteile im Magen-Darmtrakt aufweisen.

Dies wird angeblich dadurch erreicht, dass die nicht flüchtige Phase des Extraktes an Polyvinylpyrrolidon in mikrodisperser Form und/oder in der Form einer festen Lösung gebunden ist.

In DE PS 44 34 170 wird behauptet, dass die Vielzahl von wirksamen Bestandteilen an Polyvinylpyrrolidon gebunden vorliegen sollen.

Diese Behauptung stützt sich auf die Messung der Freisetzung der Dianthrone aus dem Extrakt gemäss den Vorschriften des DAB 10 V.5.4.

Gemäss den Beispielen von DE PS 44 34 170 setzt sich aber der Extrakt nur aus etwa 0,03 % (0,1 mg) Dianthronen und etwa 99,97 % (289,9 mg) nicht explizit genannten weiteren Wirkstoffen, Begleitstoffen und Polyvinylpyrrolidon zusammen.

Experimentelle Angaben zur Bioverfügbarkeit der übrigen etwa 99 % der weiteren wirksamen Bestandteile und Begleitstoffe sind nicht vorhanden.

Somit wird kein experimenteller Beweis für die obige Behauptung geliefert.

Beim in DE PS 44 34 170 beschriebenen Verfahren handelt es sich bis zum Erhalt des Fluidextraktes um ein weiter oben schon genanntes herkömmliches Verfahren, mit welchem keine vollständige Extraktion der Wirkstoffe erreicht wird.

Das Polyvinylpyrrolidon wird ausschliesslich zu diesem Fluidextrakt hinzugegeben.

In EP 0 599 307 A1 wird ebenfalls ein herkömmliches Verfahren zur Herstellung eines Fluidextraktes von Hypericum perforatum L. beschrieben.

Die in diesem Fluidextrakt enthaltenen Dianthrone lassen sich mittels der Hinzugabe von Polyvinylpyrrolidon selektiv mittels Ausfällung und Filtration aus diesem Fluidextrakt entfernen.

Im so erhaltenen nahezu Dianthron-freien Produkt werden weitere aktive Bestandteile experimentell nachgewiesen.

Somit besteht ein Widerspruch zwischen den in DE PS 44 34 170 und EP 0 599 307 A1 enthaltenen Aussagen.

In JP-A-60 222 412 wird ein Verfahren zur Abtrennung von unlöslichen Substanzen, welche in wässrigen flüssigen Arzneimitteln enthalten sind, beschrieben.

Diese Arzneimittel enthalten Pflanzeninhaltsstoffe.

Gemäss diesem Dokument können rohe Arzneimittel-Substanzen auf der Basis von Pflanzenextrakten unlösliche Mikropartikel enthalten, welche oft nur schwierig mit üblichen Filtrationsmethoden abgetrennt werden können, und welche Trübungen und Ausfällungen verursachen können.

Gemäss diesem Dokument ist gefunden worden, dass einige aus Pflanzen extrahierte rohe Arzneimittel-Substanzen zusammen mit Polyvinylpyrrolidon weisse Trübungen bilden können.

Wenn aber vorgängig der Hinzugabe von Polyvinylpyrrolidon ein mit Polyoxyethylen gehärtetes Rizinusöl-Derivat hinzugegeben wird, dann aggregieren Substanzen und bilden leicht Prezipitate, welche auf einfache Weise abgetrennt werden können.

Das heisst, dass gemäss diesem Dokument solche Substanzen vorgängig entfernt werden, welche Trübungen und Ausfällungen bilden. Die aggregatartigen Ausfällungen werden nicht näher beschrieben.

Ausgangsmaterial ist immer ein bereits hergestellter Pflanzenextrakt.

Gemäss diesem Dokument wurde also die unerwünschte Niederschlagsbildung optimiert durch vorgängige Hinzugabe eines mit Polyoxyethylen gehärteten Rizinusöl-Derivates, was zu aggregatartigen und damit besser separierbaren Ausfällungen führt.

Es ist ein Ziel der vorliegenden Erfindung, Extrakte aus Pflanzen oder Teilen davon und diese Extrakte enthaltende pharmazeutisch akzeptable Darreichungsformen zur Verfügung zu stellen, wobei diese Extrakte das jeweils gewünschte genuine Substanzgemisch in vollständiger oder nahezu vollständiger Form enthalten sollen.

Diese Extrakte und Darreichungsformen sollen stabil und homogen sein, und insbesondere die entsprechenden in den internationalen ICH-Richtlinien enthaltenen Vorgaben erfüllen.

Diese Extrakte und Darreichungsformen sollen im Gegensatz zu herkömmlichen Produkten einen wesentlich reduzierten oder keinen Gehalt an Folgeprodukten aufweisen.

Diese Extrakte und Darreichungsformen sollen mittels einem einfachen und wirtschaftlichen Verfahren hergestellt werden können.

Völlig überraschend wurde gefunden, dass die obigen Ziele erreicht werden, wenn man während oder nach der Extraktion der Pflanze oder eines Teiles davon ein in den Patentansprüchen definiertes Mittel A hinzugibt.

Die Erfindung ist durch die Merkmale in den unabhängigen Ansprüchen definiert.

Bevorzugte Ausführungsformen sind in den abhängigen Ansprüchen definiert.

Im erfindungsgemässen Verfahren werden als Mittel A vorzugsweise Proteinhydrolysate und/oder Polyvinylpyrrolidone verwendet.

Der Extrakt von Allium sativum L. wird vorzugsweise bis zum Erhalt des Fluidextraktes gemäss dem in EP 0 347 493 beschriebenen Verfahren hergestellt.

Es wurde festgestellt, dass die Hinzugabe eines Mittels A, beispielsweise Kollidon K 25 (BASF), zu diesem Fluidextrakt, gefolgt von der anschliessenden Weiterverarbeitung zu einem Trockenextrakt, ein Produkt ergibt, das sich durch eine signifikant erniedrigte Empfindlichkeit gegenüber erhöhten Temperaturen auszeichnet.

Lagert man herkömmliche Knoblauchzubereitungen bei erhöhten Temperaturen, so werden - wie oben schon erwähnt - bereits bei Temperaturen von mehr als 25°C Verluste an aktiven Verbindungen beobachtet.

Diese Verluste werden selbst bei einer Temperatur von 30°C bei einer erfindungsgemässen Knoblauchzubereitung nicht beobachtet.

Diese Stabilitätsverbesserung stellt einen grossen Fortschritt in zweifacher Hinsicht dar.

So werden einerseits Kosten beim Transport und bei der Lagerung reduziert, und andererseits wird die Sicherheit des Arzneimittels erhöht.

Es wurde festgestellt, dass die Hinzugabe eines Mittels A, beispielsweise Kollidon 17 PF (BASF), zu einem hauptsächlich wasserlösliche Verbindungen enthaltenden Fluidextrakt von Melilotus officinalis L. Lam. em. Thuill., gefolgt von der anschliessenden Weiterverarbeitung zu einem Trockenextrakt, ein Produkt ergibt, das sich durch einen signifikant erhöhten Gehalt an Cumarinen auszeichnet.

Dies erklärt sich dadurch, dass die normalerweise bei der Verdampfung des Wasser/Ethanol-Gemisches flüchtigen Cumarine offenbar durch eine Wechselwirkung mit dem Mittel A zurückgehalten werden und so im Rückstand bleiben.

Dies stellt einen grossen Fortschritt dar, weil es bisher nicht möglich war, wässrig/ethanolische, Cumarine enthaltende Lösungen ohne den Verlust der Cumarine in wirtschaftlicher Weise zu konzentrieren.

Von besonderer Bedeutung sind erfindungsgemässe Extrakte von Melilotus, weil sie sich für die Herstellung von injizierbaren Präparaten wesentlich besser als herkömmliche Produkte eignen, weil sie die gewünschten wasserlöslichen aktiven Verbindungen und keine Wachse, Chlorophyll und weitere schwer wasserlösliche Verbindungen/Rückstände enthalten.

Herkömmliche, Cumarine enthaltende Extrakte sind nur unter Verwendung hochlipophiler, wasserfreier oder nahezu wasserfreier Lösungsmittel erhältlich.

Deshalb enthalten herkömmliche Extrakte weniger gewünschte wasserlösliche aktive Verbindungen und dafür mehr für die Wirkung und für die Verwendung hinderliche und unerwünschte Verbindungen.

Es wurde festgestellt, dass die Hinzugabe eines Mittels A, beispielsweise Kollidon K 90 (BASF) zu einem Fluidextrakt von Chelidonium majus L., gefolgt von der anschliessenden Weiterverarbeitung zu einem Trokkenextrakt, ein Produkt ergibt, das sich durch einen signifikant erhöhten Gehalt an Alkaloiden sowie durch eine Homogenität auszeichnet.

Der hohe Gehalt an Alkaloiden lässt sich dadurch erklären, dass offenbar durch eine Wechselwirkung zwischen den Alkaloiden und dem Mittel A die bei herkömmlich hergestellten Produkten festgestellten Zersetzungsprozesse verhindert werden.

Die Homogenität dieses erfindungsgemässen Extraktes lässt sich dadurch erklären, dass offenbar durch eine Wechselwirkung zwischen dem Mittel A und den Isoprenoiden eine Polymerisation Letzterer zu Latex und/oder Latex-ähnlichen Verbindungen unterbleibt, und damit keine Phasentrennung auftritt.

Sowohl der gegenüber herkömmlichen Zubereitungen erhöhte Gehalt an Alkaloiden als auch die Homogenität des Produktes stellen grosse Fortschritte dar.

Der Vermeidung der Zersetzung der Alkaloide kommt eine besondere Bedeutung zu, weil alle wichtigen Chelidoniumalkaloide Methylendioxy-Gruppen enthalten, die bei Zersetzungsreaktionen als Formaldehyd abgespalten werden können.

Bei solchen Produkten kann nicht ausgeschlossen werden, dass die Zersetzungsprodukte toxischer Natur sind.

Die nachfolgenden Beispiele sollen die vorliegende Erfindung illustrieren.

### Beispiel 1

400 kg Herba Chelidonii (Chelidonium majus L.) mit einem gemessenen Gesamtalkaloidgehalt von 1,0 Gew.-% wurden in getrockneter und geschnittener Form (Schnittgrösse 1 bis 3 cm) mit 3200 kg eines Gemisches aus 7 Gewichtsteilen Ethanol und 3 Gewichtsteilen Wasser versetzt.

Dieses Gemisch wurde in einem Behältnis mit Schneid-, Rühr-, Heiz- und Kühlvorrichtungen bei Raumtemperatur während 30 Minuten unter weiterer Zerkleinerung der Pflanzenteile gerührt.

Anschliessend wurde auf eine Temperatur zwischen 60°C und 70°C erwärmt. Bei dieser Temperatur wurde unter Rühren während 2 Stunden extrahiert.

Anschliessend wurde filtriert. Zum erhaltenen Filtrat wurden 15 kg Kollidon K 90 (BASF) hinzugegeben und unter Rühren bei einer Temperatur zwischen 40°C und 50°C gelöst.

Die erhaltene Lösung wurde bei einem Druck zwischen 50 mbar und 100 mbar bei einer Temperatur zwischen 40°C und 50°C bis zu einem Trockensubstanzgehalt von 40 Gew.-% eingeengt.

Es wurden 220 kg Spissumextrakt mit einem gemessenen Gesamtalkaloidgehalt (DAB) von 1,82 Gew.-% erhalten.

Dieser Gehalt entspricht einer 100%-igen Ausbeute an Alkaloiden.

### Beispiel 2

220 kg des gemäss Beispiel 1 erhaltenen Spissumextraktes wurden mit 12 kg gefällter Kieselsäure (DAB) homogen vermischt.

Dieses Gemisch wurde bei einem Druck zwischen 20 mbar und 30 mbar und einer maximalen Produkttemperatur von 40°C während 90 Minuten getrocknet.

Der so erhaltene Trockenextrakt wurde gesiebt und homogen gemischt.

Man erhielt 100 kg Trockenextrakt mit einem gemessenen Gesamtalkaloidgehalt (DAB) von 3,93 Gew.-%.

Dieser Gehalt entspricht einer 98%-igen Ausbeute an Alkaloiden.

### Beispiel 3

Es wurden 4 kg Herba Chelidonii, 32 kg eines Gemisches aus 7 Gewichtsteilen Ethanol und 3 Gewichtsteilen Wasser sowie 0,15 kg Kollidon K 90 gemäss Beispiel 1 verarbeitet.

Im erhaltenen Spissumextrakt (2,0 kg) wurde ein Gesamtalkaloidgehalt (DAB) von 2,0 Gew.-% gemessen.

Dieser Gehalt entspricht einer 100 %-igen Ausbeute an Alkaloiden.

2,0 kg dieses Spissumextraktes wurden mit 120 g gefällter Kieselsäure (DAB) homogen vermischt und gemäss Beispiel 2 verarbeitet.

Im erhaltenen Trockenextrakt (1 kg) wurde ein Gesamtalkaloidgehalt (DAB) von 4,08 Gew.-% gemessen.

Dieser Gehalt entspricht einer 102 %-igen Ausbeute an Alkaloiden.

Dem Fachmann ist bekannt, dass bei der angewendeten Messmethode aufgrund der Messgenauigkeit Werte von mehr als 100 % auftreten können.

### Beispiel 4 (Vergleichsbeispiel)

Es wurden 4 kg Herba Chelidonii und 32 kg eines Gemisches aus 7 Gewichtsteilen Ethanol und 3 Gewichtsteilen Wasser ohne die Hinzugabe von Kollidon K 90 gemäss Beispiel 1 verarbeitet.

Im erhaltenen Spissumextrakt (1,5 kg) wurde ein Gesamtalkaloidgehalt (DAB) von 1,7 Gew.-% gemessen.

Dieser Gehalt entspricht einer 64 %-igen Ausbeute an Alkaloiden.

1,5 kg dieses Spissumextraktes wurden mit 120 g gefällter Kieselsäure (DAB) homogen vermischt und gemäss Beispiel 2 verarbeitet.

Im erhaltenen Trockenextrakt (0,66 kg) wurde ein Gesamtalkaloidgehalt (DAB) von 2,3 Gew.-% gemessen.

Dieser Gehalt entspricht einer 57,5 %-igen Ausbeute an Alkaloiden.

In den folgenden Tabellen 1 und 2 sind verschiedene Daten zusammengefasst.

**Tabelle 1**

| | Beispiel 3 (Spissumextrakt) | Beispiel 4 (Spissumextrakt) |
|---|---|---|
| Ausbeute (gemessen als Trockensubstanz und auf die Menge der eingesetzten Pflanze bezogen) | 17,5 % *) | 11 % |
| | | |
| Ausbeute an Alkaloiden | 100 % | 64 % |
| | | |
| Viskosität (Pa.s, 25°C) | 3,0 | 1,0 bis 50,0 **) |
| | | |
| Sedimentation | nein | ja |
| | | |
| Flotation | nein | ja |
| | | |
| Homogenität | ja | nein |

| | | |
|---|---|---|
| *) ohne Kollidon K 90 | | |
| **) inhomogenes, mehrphasiges Gemisch | | |

**Tabelle 2**

| | Beispiel 3 (Trockenextrakt) | Beispiel 4 (Trockenextrakt) |
|---|---|---|
| Ausbeute (gemessen als Trockensubstanz und auf die Menge der eingesetzten Pflanze bezogen) | 17,5 % *) | 11 % **) |
| | | |
| Ausbeute an Alkaloiden | 102 % | 57,5 % |
| | | |
| Homogenität | ja | nein |
| | | |
| Sinterpunkt | 71°C | 43°C |

| | | |
|---|---|---|
| *) ohne Kollidon K 90 und ohne gefällte Kieselsäure | | |
| **) ohne gefällte Kieselsäure | | |

### Beispiel 5

4 kg Herba Meliloti (Melilotus officinalis L. Lam. em. Thuill.) mit einem gemessenen Cumaringehalt von 0,38 Gew.-% (HPLC) wurden in getrockneter und geschnittener Form (Schnittgrösse 1 bis 3 cm) mit 60 kg eines Gemisches aus 1 Gewichtsteil Ethanol und 3 Gewichtsteilen Wasser versetzt.

Dieses Gemisch wurde in einem Behältnis mit Schneid-, Rühr-, Heiz- und Kühlvorrichtungen bei einer Temperatur von 30°C bis 40°C während 2 Stunden unter weiterer Zerkleinerung der Pflanzenteile gerührt und extrahiert.

Anschliessend wurde filtriert. Zum erhaltenen Filtrat (54 kg, Trockensubstanzgehalt 1,5 Gew.-%, 1,56 Gew.-% Cumaringehalt, bezogen auf die Trockensubstanz) wurden 200 g Kollidon 17 PF (BASF) hinzugegeben und unter Rühren bei einer Temperatur zwischen 30°C und 40°C gelöst.

Die erhaltene Lösung wurde bei einem Druck zwischen 20 mbar und 30 mbar bei einer Temperatur zwischen 30°C und 40°C bis zu einem Trockensubstanzgehalt von 30 Gew.-% eingeengt.

Es wurden 3,3 kg Spissumextrakt mit einem gemessenen Cumaringehalt (HPLC) von 1,55 Gew.-%, bezogen auf die Trockensubstanz, erhalten.

Dieser Gehalt entspricht einer 99 %-igen Ausbeute an Cumarinen.

### Beispiel 6

3,3 kg des gemäss Beispiel 5 erhaltenen Spissumextraktes wurden sprühgetrocknet.

Der so erhaltene Trockenextrakt wurde gesiebt und homogen gemischt.

Man erhielt 1 kg Trockenextrakt mit einem gemessenen Cumaringehalt (HPLC) von 1,48 Gew.-%.

Dieser Gehalt entspricht einer 94,9%-igen Ausbeute an Cumarinen.

### Beispiel 7 (Vergleichsbeispiel)

4 kg Herba Meliloti und 60 kg eines Gemisches aus 1 Gewichtsteil Ethanol und 3 Gewichtsteilen Wasser wurden ohne die Hinzugabe von Kollidon 17 PF gemäss Beispiel 5 verarbeitet.

Im erhaltenen Filtrat wurden die gleichen Werte wie in Beispiel 5 gemessen.

Im erhaltenen Spissumextrakt (2,7 kg) wurde ein Cumaringehalt (HPLC) von 0,93 Gew.-%, bezogen auf die Trockensubstanz, gemessen.

Dieser Gehalt entspricht einer 48,4%-igen Ausbeute an Cumarinen.

2,7 kg dieses Spissumextraktes wurden gemäss Beispiel 6 zu einem Trockenextrakt verarbeitet.

Man erhielt 800 g Trockenextrakt mit einem gemessenen Cumaringehalt (HPLC) von 0,90 Gew.-%.

Dieser Gehalt entspricht einer 46,5%-igen Ausbeute an Cumarinen.

### Beispiel 8

12 kg frische ganze Bulbus Allii sativi (Allium sativum L.) wurden mit 84 kg eines Gemisches aus 9 Gewichtsteilen Ethanol und 1 Gewichtsteil Wasser versetzt.

Dieses Gemisch wurde in einem Behältnis mit Schneid-, Rühr-, Heiz- und Kühlvorrichtungen bei Raumtemperatur während 10 Minuten unter Zerkleinerung der Pflanzenteile gerührt.

Dieses Gemisch wurde bei Raumtemperatur während 80 Minuten gerührt und extrahiert.

Anschliessend wurde filtriert. Zum erhaltenen Filtrat wurden 300 g Kollidon K 25 (BASF) hinzugegeben und unter Rühren bei Raumtemperatur gelöst.

Die erhaltene Lösung wurde bei einem Druck zwischen 30 mbar und 100 mbar und bei einer Temperatur zwischen 30°C und 40°C bis zu einem Trockensubstanzgehalt von 60 Gew.-% eingeengt.

Es wurden 1,7 kg Spissumextrakt erhalten.

Dieser Spissumextrakt wurde bei einem Druck zwischen 20 mbar und 30 mbar und einer maximalen Produkttemperatur von 40°C während 90 Minuten getrocknet.

Der so erhaltene Trockenextrakt wurde gesiebt und homogen gemischt.

Man erhielt 1 kg Trockenextrakt mit einem gemessenen Alliingehalt (HPLC) von 9,0 Gew.-%.

### Beispiel 9 (Vergleichsbeispiel)

Es wurden 12 kg frische ganze Bulbus Allii sativi und 84 kg eines Gemisches aus 9 Gewichtsteilen Ethanol und 1 Gewichtsteil Wasser ohne die Hinzugabe von Kollidon K 25 gemäss Beispiel 8 verarbeitet.

Im erhaltenen Trockenextrakt (700 g) wurde ein Alliingehalt (HPLC) von 12,1 Gew.-% gemessen.

In der folgenden Tabelle 3 sind verschiedene Daten betreffend die Lagerstabilitäten zusammengefasst.

**Tabelle 3**

| | Beispiel 8 (Trockenextrakt) | | | Beispiel 9 (Trockenextrakt) | | |
|---|---|---|---|---|---|---|
| Temperatur (°C) | 20 | 25 | 30 | 20 | 25 | 30 |
| | | | | | | |
| Alliingehalt (in %, bezogen auf den als 100% gesetzten Anfangswert) am Anfang | 100 | 100 | 100 | 100 | 100 | 100 |
| | | | | | | |
| nach 1 Monat | 100 | 100 | 100 | 100 | 98 | 85 |
| nach 3 Monaten | 100 | 100 | 100 | 100 | 97 | 64 |
| nach 6 Monaten | 103 | 103 | 98 | 100 | 97 | 31 |
| nach 12 Monaten | *) | *) | *) | 98 | 95,7 | 26 |
| nach 18 Monaten | *) | *) | *) | 95 | 94 | 13 |
| nach 24 Monaten | *) | *) | *) | 94 | 93 | 9 |
| nach 36 Monaten | *) | *) | *) | 92 | 92 | 3 |

| | | | | | | |
|---|---|---|---|---|---|---|
| *) nicht gemessen | | | | | | |

## Patentansprüche

1. Verfahren zur Herstellung eines stabilen, homogenen, von Folgeprodukten freien oder nahezu freien Extraktes, welcher das jeweils gewünschte genuine Substanzgemisch in vollständiger oder nahezu vollständiger Form enthält, wobei dieser Extrakt aus Pflanzen oder Teilen davon erhalten wird, welche im frischen und/oder getrockneten Zustand in bearbeiteter oder unbearbeiteter Form eingesetzt werden können, **dadurch gekennzeichnet, dass** man
- in einem ersten Schritt
entweder gemäss einer ersten Variante
- A.) 1.) in beliebiger Reihenfolge die genannten Pflanzen oder Teile davon mit wenigstens einem Lösungsmittel und wenigstens einem Mittel, nachfolgend als Mittel A bezeichnet, vermischt, löst und anschliessend extrahiert, wobei dieses Mittel A durch seine Anwesenheit
- die Löslichkeit von wenigstens einer im Extrakt enthaltenen Substanz erhöht,
- die Viskosität des Extraktes in Anwesenheit eines Lösungsmittels oder Lösungsmittelgemisches erhöht,
- den Dampfdruck der im Extrakt gegebenenfalls enthaltenen flüchtigen Substanzen beeinflusst,
- chemische und/oder enzymatische Reaktionen zwischen einer oder mehrerer im Extrakt enthaltenen Substanz(en) verhindert oder zumindest wesentlich reduziert,
- durch Wechselwirkung mit einer oder mehrerer im Extrakt enthaltenen Substanz(en) deren Sedimentation und/oder Flotation verhindert oder zumindest wesentlich reduziert, wenn der Extrakt in flüssiger, halbfester oder in wenigstens einem Lösungsmittel gelöster und/oder suspendierter und/oder emulgierter Form vorliegt, und
- die Stabilität und/oder die Homogenität wenigstens einer im Extrakt enthaltenen Substanz erhöht,
und
- A.)2.) den so erhaltenen Extrakt von den unlöslichen Stoffen abtrennt,
oder gemäss einer zweiten Variante
- B.)1.) die genannten Pflanzen oder Teile davon mit wenigstens einem Lösungsmittel vermischt und extrahiert,
- B.)2.) den so erhaltenen Extrakt von den unlöslichen Stoffen abtrennt, und
- B.)3.) diesen Extrakt mit wenigstens einem oben genannten Mittel A vermischt, wobei sich das Mittel A im Extrakt löst, und
- in einem zweiten Schritt
den gemäss dem ersten Schritt erhaltenen Extrakt, in welchem das Mittel A enthalten ist und enthalten bleibt, gewinnt, wobei die Pflanzen ausgewählt sind aus der Gruppe, bestehend aus:
Allium-Arten (z.B. A. cepa L., A. ursinum L., A. sativum L.: Bulbus)
Ammi visnaga L.
Arnica montana L.
Asparagus officinalis L.
Astragalus-Arten
Avena sativa L.
Berberis vulgaris L.
Brassica nigra L. Koch
Calendula officinalis L.
Capsicum frutescens L.
Carum carvi L.
Chelidonium majus L.
Chrysanthemum parthenium
Chrysanthemum vulgare Asch.
Cimicifuga racemosa L.
Centella asiatica L.
Cucurbita pepo L.
Curcuma-Arten
Ephedra sinica (Stapf) und andere Arten
Euphrasia offic. L.
Foeniculum vulgare Miller
Fumaria officinalis L.
Glycine max. L.
Ilex paraguariensis St. Hil.
Jasminum grandiflorum L.
Laurus nobilis L.
Matricaria chamomilla L.
Melilotus officinalis L. Lam.em. Thuill.
Olea europaea L.
Orthosiphon stamineus Benth.
Panax ginseng Meyer
Petroselinum crispum (Mill.) Nym.
Peumus boldus (Molina)
Phaseolus vulgaris L.
Pimenta dioica L. Merill
Pimpinella anisum L.
Plantago lanceolata L.
Rauvolfia-Arten
Salix alba L. und alle Arten
Sanguinaria canadensis L.
Schisandra chinensis Baill.
Smilax-Arten
Sylibum marianum L. Gaertner
Taraxacum officinale Web.
Urtica dioica L.
Valeriana officinalis L. und ihre Varietäten
Vitex agnus castus L.
Zingiber officinale Roscoe.

2. Verfahren nach Anspruch 1, **dadurch gekennzeichnet, dass** man den am Ende des zweiten Schrittes erhaltenen Extrakt zu einem Spissumextrakt einengt, insbesondere bei einer Temperatur von unterhalb 70°C, wobei sich der jeweils angewendete Druck nach dem(den) verwendeten Lösungsmittel(n) richtet, und gegebenenfalls
- entweder den so erhaltenen Spissumextrakt trocknet, vorzugsweise mittels einer Vakuumband-Vorrichtung, und so einen Trockenextrakt erhält, der bei einer Temperatur von unterhalb 60°C in festem Aggregatzustand ist,
- oder den so erhaltenen Spissumextrakt mit wenigstens einem pharmazeutisch akzeptablen Lösungsmittel vermischt, insbesondere ausgewählt aus der Gruppe, bestehend aus Wasser, Glycerol, Propylenglykol, Polyethylenglykolen mit einem mittleren Molekulargewicht im Bereich von 300 bis 1500, insbesondere 300, und so eine Zubereitung erhält, die bei einer Temperatur von unterhalb 50°C in flüssigem Aggregatzustand ist.

3. Verfahren nach einem der Ansprüche 1 bis 2, **dadurch gekennzeichnet, dass** man dem Gemisch zu einem beliebigen Zeitpunkt, vorzugsweise am Ende des zweiten Schrittes oder vor dem Trocknen oder vor dem Vermischen mit einem pharmazeutisch akzeptablen Lösungsmittel, noch wenigstens einen pharmazeutisch akzeptablen Zusatzund/oder Hilfsstoff hinzufügt, insbesondere ausgewählt aus der Gruppe, bestehend aus Emulgatoren, Stabilisatoren, Antioxidantien, Farbstoffen, Aromen, Sprengmitteln, Mitteln, welche die Rieselfähigkeit günstig beeinflussen, Mitteln, welche die Kompaktierbarkeit günstig beeinflussen, Mitteln, welche den Schmelzpunkt erhöhen, und Mitteln, welche die Hygroskopizität reduzieren.

4. Verfahren nach Anspruch 1, **dadurch gekennzeichnet, dass** man
- in einem dritten Schritt den am Ende des zweiten Schrittes erhaltenen Extrakt mit wenigstens einem Trägermaterial für den genannten Extrakt vermischt, wobei dieses Trägermaterial verkapselbar und gegenüber allen im so erhaltenen Gemisch vorhandenen Substanzen inert sein muss, und
- in einem vierten Schritt das so erhaltene Gemisch teilweise oder vollständig vom Lösungsmittel oder Lösungsmittelgemisch befreit.

5. Verfahren nach Anspruch 4, **dadurch gekennzeichnet, dass** das Trägermaterial ausgewählt ist aus der Gruppe, bestehend aus
- Polyethylenglykolen mit einem mittleren Molekulargewicht im Bereich von 300 bis 600, insbesondere 300,
- Fettsäureestern von Polyglycerinen,
- Lecithinen,
- Silikonölen,
- Sorbitanfettsäureestern,
- Sorbaten von Fettsäuren,
- Polysorbaten von Fettsäuren,
- Wachsen,
- Polyglycerinen,
- Triglyceriden,
- Fettsäuren,
- fetten Oelen, und
- Paraffinen,
einschliesslich beliebige Gemische davon.

6. Verfahren nach einem der Ansprüche 4 bis 5, **dadurch gekennzeichnet, dass** man dem am Ende des vierten Schrittes erhaltenen Gemisches soviel Wasser und/oder Glycerol und/oder Propylenglykol hinzufügt, dass ein entsprechender Gehalt davon von 5 Gew.-% bis 20 Gew.-%, insbesondere von 12 Gew.-% bis 15 Gew.-%, resultiert.

7. Verfahren nach einem der Ansprüche 1 bis 6 **dadurch gekennzeichnet, dass** das Lösungsmittel zur Herstellung des Pflanzenextraktes ein polares Lösungsmittel ist, insbesondere ausgewählt aus der Gruppe, bestehend aus
- Wasser,
- Alkoholen, insbesondere einem C₁- bis C₄-Alkohol, vorzugsweise Ethanol, Glycerol oder Propylenglykol,
- Ketonen, insbesondere einem C₃- bis C₅-Keton, vorzugsweise Aceton, und
- Estern, insbesondere einem Alkylacetat, wobei der Alkylrest vorzugsweise 1 bis 4 C-Atome hat,
einschliesslich beliebige Mischungen davon, beispielsweise ein Gemisch aus Wasser und Ethanol.

8. Verfahren nach einem der Ansprüche 1 bis 7, **dadurch gekennzeichnet, dass** das Mittel A eine polymere Verbindung ist, und insbesondere ausgewählt ist aus der Gruppe, bestehend aus
- Polyvinylpyrrolidonen, abgekürzt mit PVP,
- Vinylacetat - Crotonsäure - Copolymeren,
- Methacrylsäure-Ethylacrylat-Copolymeren,
- Blockcopolymeren aus Polyethylenglykolen und Polypropylenglykolen,
- Proteinen und Proteinhydrolysaten aus Eiweissen pflanzlichen und/oder tierischen Ursprungs, beispielsweise Gelatinen, und
- Blockcopolymeren aus Ethylenoxyd und Propylenoxyd,
einschliesslich beliebige Mischungen davon.

9. Verfahren nach einem der Ansprüche 1 bis 8, **dadurch gekennzeichnet, dass**
- das Gewichtsverhältnis von eingesetzter Pflanze zu verwendetem Extraktionsmittel von 1:1 bis 1:30, insbesondere von 1:5 bis 1:15, vorzugsweise von 1:4 bis 1:8, beträgt,
- das Gewichtsverhältnis von eingesetzter Pflanze zu verwendetem Mittel A im Falle von
-- PVP von 5:1 bis 120:1,
-- Vinylacetat-Crotonsäure-Copolymeren von 10:1 bis 50:1,
-- Methacrylsäure-Ethylacrylat-Copolymeren von 10:1 bis 50:1,
-- Blockcopolymeren aus Polyethylenglykolen und Polypropylenglykolen von 2:1 bis 50:1,
-- Proteinen und Proteinhydrolysaten aus Eiweissen pflanzlichen und/oder tierischen Ursprungs von 2:1 bis 50:1,
-- Blockcopolymeren aus Ethylenoxyd und Propylenoxyd von 2:1 bis 50:1,
beträgt.

10. Verfahren nach einem der Ansprüche 1 bis 9, **dadurch gekennzeichnet, dass** man eine Pflanze oder Teile davon aus der Familie der Papaveraceen, beispielsweise Chelidonium majus L., Fumaria officinalis L., Sanguinaria canadensis L., mit einem Wasser/Alkohol-Gemisch, insbesondere ein Gemisch aus Wasser und Ethanol, vermischt und extrahiert, den so erhaltenen Extrakt von den unlöslichen Stoffen abtrennt, die so erhaltene Lösung mit einem PVP vermischt, insbesondere ein PVP mit einem mittleren Molekulargewicht im Bereich von 1'000'000 bis 1'500'000, wobei das Gewichtsverhältnis von eingesetzter Pflanze zu verwendetem PVP von 10:1 bis 60:1 beträgt, und gegebenenfalls das so erhaltene Gemisch zu einem Spissumextrakt mit einem Wassergehalt von 55 Gew.-% bis 75 Gew.-%, bezogen auf den Spissumextrakt, einengt, insbesondere bei einer Temperatur von unterhalb 70°C und einem Druck von weniger als 200 mbar, zum so erhaltenen Gemisch ein Mittel hinzugibt, welches den Schmelzpunkt erhöht, beispielsweise Kieselsäure, vorzugsweise gefällte Kieselsäure, und dieses Gemisch trocknet.

11. Verfahren nach einem der Ansprüche 1 bis 9, **dadurch gekennzeichnet, dass** man eine Pflanze oder Teile davon aus der Familie der Fabaceen, beispielsweise Melilotus officinalis L. Lam. Em. Thuill., Phaseolus vulgaris L., Astragalus-Arten, mit einem Wasser/Alkohol-Gemisch, insbesondere ein Gemisch aus Wasser und Ethanol, vermischt und extrahiert, den so erhaltenen Extrakt von den unlöslichen Stoffen abtrennt, die so erhaltene Lösung mit einem PVP vermischt, insbesondere ein PVP mit einem mittleren Molekulargewicht im Bereich von 2'000 bis 54'000, insbesondere 7'000 bis 11'000, wobei das Gewichtsverhältnis von eingesetzter Pflanze zu verwendetem PVP von 10:1 bis 40:1 beträgt, und gegebenenfalls das so erhaltene Gemisch zu einem Spissumextrakt mit einem Wassergehalt von 40 Gew.-% bis 70 Gew.-%, bezogen auf den Spissumextrakt, einengt, insbesondere bei einer Temperatur von unterhalb 50°C und einem Druck von weniger als 80 mbar, vorzugsweise bei einer Temperatur von 30°C bis 40°C und einem Druck von weniger als 50 mbar, und dieses Gemisch trocknet.

12. Verfahren nach einem der Ansprüche 1 bis 9, **dadurch gekennzeichnet, dass** man eine Pflanze oder Teile davon aus der Familie der Liliaceen, beispielsweise Allium-Arten (z.B. A. cepa L., A. ursinum L., A. sativum L. : Bulbus), mit einem Wasser/Alkohol-Gemisch, insbesondere ein Gemisch aus Wasser und Ethanol, vermischt und extrahiert, den so erhaltenen Extrakt von den unlöslichen Stoffen abtrennt, die so erhaltene Lösung mit einem PVP vermischt, insbesondere ein PVP mit einem mittleren Molekulargewicht im Bereich von 2'000 bis 54'000, insbesondere 28'000 bis 34'000, wobei das Gewichtsverhältnis von eingesetzter Pflanze zu verwendetem PVP von 20:1 bis 120:1, insbesondere 90:1, beträgt, und gegebenenfalls das so erhaltene Gemisch zu einem Spissumextrakt mit einem Wassergehalt von 30 Gew.-% bis 60 Gew.-%, bezogen auf den Spissumextrakt, einengt, insbesondere bei einer Temperatur von unterhalb 70°C und einem Druck von weniger als 200 mbar, zum so erhaltenen Gemisch gegebenenfalls ein Antioxidanz hinzugibt, beispielsweise α-Tocopherol, und dieses Gemisch trocknet.

13. Verwendung von polymeren Verbindungen, insbesondere ausgewählt aus der Gruppe, bestehend aus
- Polyvinylpyrrolidonen, abgekürzt mit PVP,
- Vinylacetat - Crotonsäure - Copolymeren,
- Methacrylsäure-Ethylacrylat-Copolymeren,
- Blockcopolymeren aus Polyethylenglykolen und Polypropylenglykolen,
- Proteinen und Proteinhydrolysaten aus Eiweissen pflanzlichen und/oder tierischen Ursprungs, beispielsweise Gelatinen, und
- Blockcopolymeren aus Ethylenoxyd und Propylenoxyd,
einschliesslich beliebige Mischungen davon,
als Mittel zur Durchführung des Verfahrens nach einem der Ansprüche 1 bis 12.

14. Stabiler, homogener, von Folgeprodukten freier oder nahezu freier Extrakt aus Pflanzen, welche aus der in Anspruch 1 genannten Gruppe ausgewählt sind, oder Teilen davon, **dadurch gekennzeichnet, dass** dieser Extrakt nebst dem jeweils gewünschten genuinen Substanzgemisch in vollständiger oder nahezu vollständiger Form noch wenigstens ein Mittel A enthält, wobei dieses Mittel A
- die Löslichkeit von wenigstens einer im Extrakt enthaltenen Substanz erhöht,
- die Viskosität des Extraktes in Anwesenheit eines Lösungsmittels oder Lösungsmittelgemisches erhöht,
- den Dampfdruck der im Extrakt gegebenenfalls enthaltenen flüchtigen Substanzen beeinflusst,
- chemische und/oder enzymatische Reaktionen zwischen einer oder mehrerer im Extrakt enthaltenen Substanz(en) verhindert oder zumindest wesentlich reduziert,
- durch Wechselwirkung mit einer oder mehrerer im Extrakt enthaltenen Substanz(en) deren Sedimentation und/oder Flotation verhindert oder zumindest wesentlich reduziert, wenn der Extrakt in flüssiger, halbfester oder in wenigstens einem Lösungsmittel gelöster und/oder suspendierter und/oder emulgierter Form vorliegt, und
- die Stabilität und/oder die Homogenität wenigstens einer im Extrakt enthaltenen Substanz erhöht,
und
in flüssiger, halbfester, fester oder in wenigstens einem Lösungsmittel gelöster und/oder suspendierter und/oder emulgierter Form vorliegt.

15. Extrakt nach Anspruch 14, **dadurch gekennzeichnet, dass** die Lösungsmittel pharmazeutisch akzeptable Lösungsmittel sind, insbesondere ausgewählt aus der Gruppe, bestehend aus Wasser, Glycerol, Propylenglykol, Polyethylenglykolen mit einem mittleren Molekulargewicht im Bereich von 300 bis 1500, insbesondere 300, wobei ein entsprechender Gehalt davon von 5 Gew.-% bis 20 Gew.-%, insbesondere von 12 Gew.-% bis 15 Gew.-%, bevorzugt ist.

16. Extrakt nach einem der Ansprüche 14 bis 15, **dadurch gekennzeichnet, dass** er noch wenigstens einen pharmazeutisch akzeptablen Zusatz- und/oder Hilfsstoff enthält, insbesondere ausgewählt aus der Gruppe, bestehend aus Emulgatoren, Stabilisatoren, Antioxidantien, Farbstoffen, Aromen, Sprengmitteln, Mitteln, welche die Rieselfähigkeit günstig beeinflussen, Mitteln, welche die Kompaktierbarkeit günstig beeinflussen, Mitteln, welche den Schmelzpunkt erhöhen, und Mitteln, welche die Hygroskopizität reduzieren.

17. Extrakt nach einem der Ansprüche 14 bis 16, **dadurch gekennzeichnet, dass** er mit wenigstens einem Trägermaterial vermischt ist, wobei dieses Trägermaterial verkapselbar und gegenüber allen im Extrakt vorhandenen Substanzen inert sein muss, und wobei das Trägermaterial vorzugsweise ausgewählt ist aus der Gruppe, bestehend aus
- Polyethylenglykolen mit einem mittleren Molekulargewicht im Bereich von 300 bis 600, insbesondere 300,
- Fettsäureestern von Polyglycerinen,
- Lecithinen,
- Silikonölen,
- Sorbitanfettsäureestern,
- Sorbaten von Fettsäuren,
- Polysorbaten von Fettsäuren,
- Wachsen,
- Polyglycerinen,
- Triglyceriden,
- Fettsäuren,
- fetten Oelen, und
- Paraffinen,
einschliesslich beliebige Gemische davon.

18. Extrakt nach einem der Ansprüche 14 bis 17, **dadurch gekennzeichnet, dass** das Mittel A eine polymere Verbindung ist, und insbesondere ausgewählt ist aus der Gruppe, bestehend aus
- Polyvinylpyrrolidonen, abgekürzt mit PVP,
- Vinylacetat - Crotonsäure - Copolymeren,
- Methacrylsäure-Ethylacrylat-Copolymeren,
- Blockcopolymeren aus Polyethylenglykolen und Polypropylenglykolen,
- Proteinen und Proteinhydrolysaten aus Eiweissen pflanzlichen und/oder tierischen Ursprungs, beispielsweise Gelatinen, und
- Blockcopolymeren aus Ethylenoxyd und Propylenoxyd,
einschliesslich beliebige Mischungen davon.

19. Extrakt nach einem der Ansprüche 14 bis 18, **dadurch gekennzeichnet, dass** das Gewichtsverhältnis von genuinem Substanzgemisch, bezogen auf die genuine Trockensubstanz, zu verwendetem Mittel A im Falle von
- PVP von 95:5 bis 70:30,
- Vinylacetat-Crotonsäure-Copolymeren von 98:2 bis 90:10,
- Methacrylsäure-Ethylacrylat-Copolymeren von 98:2 bis 90:10,
- Blockcopolymeren aus Polyethylenglykolen und Polypropylenglykolen von 98:2 bis 90:10,
- Proteinen und Proteinhydrolysaten aus Eiweissen pflanzlichen und/oder tierischen Ursprungs von 98:2 bis 70:30,
- Blockcopolymeren aus Ethylenoxyd und Propylenoxyd von 98:2 bis 90:10,
beträgt.

20. Extrakt nach einem der Ansprüche 14 bis 19, **dadurch gekennzeichnet, dass** die zur Herstellung des genuinen Substanzgemisches verwendeten Pflanzen aus der in Anspruch 1 genannten Gruppe ausgewählt sind, wobei die Familien der Papaveraceen, beispielsweise Chelidonium majus L., Fumaria officinalis L., Sanguinaria canadensis L., Fabaceen, beispielsweise Melilotus officinalis L. Lam. em. Thuill., Phaseolus vulgaris L., Astragalus-Arten, und Liliaceen, beispielsweise Allium-Arten (z.B. A.cepa L., A. ursinum L., A. sativum L.: Bulbus), bevorzugt sind.

21. Extrakt nach einem der Ansprüche 14 bis 20, **dadurch gekennzeichnet, dass** er in einer pharmazeutisch akzeptablen Darreichungsform enthalten ist, insbesondere in
- festen Darreichungsformen zur oralen Applikation, insbesondere in der Form einer Tablette, einer Filmtablette, eines Dragees, eines Pellets, einer Hartgelatine-Kapsel, einer Weichgelatine-Kapsel,
- flüssigen Darreichungsformen zur oralen, parenteralen, rektalen, vaginalen und topischen Applikation, insbesondere in der Form einer Tropflösung, eines Sprays, einer Injektionslösung, eines Sirups,
- halbfesten Darreichungsformen zur topischen, oralen, rektalen und vaginalen Applikation, insbesondere in der Form einer Crème, eines Gels, einer Salbe, einer Paste, eines Suppositoriums.

22. Extrakt nach einem der Ansprüche 14 bis 21, **dadurch gekennzeichnet, dass** die zur Herstellung des genuinen Substanzgemisches verwendete Pflanze Chelidonium majus L. ist, und dass das Mittel A ein PVP mit einem mittleren Molekulargewicht im Bereich von 1'000'000 bis 1'500'000 ist.

23. Extrakt nach einem der Ansprüche 14 bis 21, **dadurch gekennzeichnet, dass** die zur Herstellung des genuinen Substanzgemisches verwendete Pflanze Allium sativum L. ist, und dass das Mittel A ein PVP mit einem mittleren Molekulargewicht im Bereich von 2'000 bis 54'000, insbesondere 28'000 bis 34'000, ist.

24. Extrakt nach einem der Ansprüche 14 bis 21, **dadurch gekennzeichnet, dass** die zur Herstellung des genuinen Substanzgemisches verwendete Pflanze ausgewählt ist aus der Gruppe, bestehend aus Ammi visnaga L., Ephedra sinica (Stapf), Melilotus officinalis L. Lam. em. Thuill. und Zingiber officinale Roscoe, und dass das Mittel A ein PVP mit einem mittleren Molekulargewicht im Bereich von 2'000 bis 54'000, insbesondere 7'000 bis 11'000, ist.

## Claims

1. A process for the preparation of a stable, homogeneous, from secondary reaction products free or nearly free extract, which contains the respective desired genuine substance mixture in a complete or nearly complete form, whereby this extract is obtained from plants or parts thereof, which may be charged in a fresh and/or dried state in treated or untreated form, **characterized in that**
- in a first step
either according to a first variety
- A.) 1.) in whatever sequence said plants or parts thereof are mixed with at least one solvent and at least one agent, in the following part denoted as agent A, and are then extracted, whereby this agent A due to its presence
- increases the solubility of at least one in the extract contained substance,
- increases the viscosity of the extract in the presence of a solvent or a mixture of solvents,
- influences the steam pressure of the in the extract occasionally contained volatile substances,
- prevents or at least reduces considerably the chemical and/or enzymatic reactions between one or more in the extract contained substance(s),
- prevents or at least reduces considerably through interaction with one or more in the extract contained substance(s) its (their) sedimentation and/or flotation, when the extract is in liquid, semisolid or in at least one solvent dissolved and/or suspended and/or emulsified form, and
- increases the stability and/or the homogeneity of at least one in the extract contained substance,
and
- A.)2.) the so obtained extract is separated from the insoluble substances,
or according to a second variety
- B.)1.) said plants or parts thereof are mixed and extracted with at least one solvent,
- B.)2.) the so obtained extract is separated from the insoluble substances, and
- B.)3.) this extract is mixed with at least one above mentioned agent A, whereby the agent A is dissolved in the extract, and
- in a second step
the according the first step obtained extract is obtained, in which the agent A is contained and remains contained, whereby the plants are selected from the group consisting of:
Allium-species (e.g. A. cepa L., A.ursinum L., A. sativum L.: Bulbus)
Ammi visnaga L.
Arnica montana L.
Asparagus officinalis L.
Astragalus-species
Avena sativa L.
Berberis vulgaris L.
Brassica nigra L. Koch
Calendula officinalis L.
Capsicum frutescens L.
Carum carvi L.
Chelidonium majus L.
Chrysanthemum parthenium
Chrysanthemum vulgare Asch.
Cimicifuga racemosa L.
Centella asiatica L.
Cucurbita pepo L.
Curcuma-species
Ephedra sinica (Stapf) and other species
Euphrasia offic. L.
Foeniculum vulgare Miller
Fumaria officinalis L.
Glycine max. L.
Ilex paraguariensis St. Hil.
Jasminum grandiflorum L.
Laurus nobilis L.
Matricaria chamomilla L.
Melilotus officinalis L. Lam.em. Thuill.
Olea europaea L.
Orthosiphon stamineus Benth.
Panax ginseng Meyer
Petroselinum crispum (Mill.) Nym.
Peumus boldus (Molina)
Phaseolus vulgaris L.
Pimenta dioica L. Merill
Pimpinella anisum L.
Plantago lanceolata L.
Rauvolfia-species
Salix alba L. and all species
Sanguinaria canadensis L.
Schisandra chinensis Baill.
Smilax-species
Sylibum marianum L. Gaertner
Taraxacum officinale Web.
Urtica dioica L.
Valeriana officinalis L. and their varieties
Vitex agnus castus L.
Zingiber officinale Roscoe.

2. The process according to claim 1, **characterized in that** the at the end of the second step obtained extract is concentrated to a spissum extract, especially at a temperature of below 70°C, whereby the respective used pressure is adjusted according to the used solvent(s), and occasionally
- either the so obtained spissum extract is dried, preferably by means of a vacuum belt device, and so a dry extract is obtained, which is at a temperature of below 60°C in a solid state of aggregation,
- or the so obtained spissum extract is mixed with at least one pharmaceutical acceptable solvent, especially selected from the group, consisting of water, glycerol, propylenglycol, polyethylene glycols with an average molecular weight in the range from 300 to 1'500, especially 300, and so a preparation is obtained, which is at a temperature of below 50°C in a liquid state of aggregation.

3. The process according to one of claims 1 to 2, **characterized in that** to the mixture at whatever moment, preferably at the end of the second step and prior to the drying or prior to the mixing with a pharmaceutical acceptable solvent, additionally at least one pharmaceutical acceptable additive and/or auxiliary agent is added, especially selected from the group, consisting of emulsifiers, stabilizers, antioxidants, dyestuffs, aromas, disintegration agents, agents, which have a favorable influence onto the free-flowing behavior, agents, which have a favorable influence onto the comprimation, agents, which increase the melting point, and agents, which reduce the hygroscopicity.

4. The process according to claim 1, **characterized in that**
- in a third step the at the end of the second step obtained extract is mixed with at least one carrier material for said extract, whereby this carrier material must be encapsulatable and must be inert against all in the so obtained mixture contained substances, and
- in a fourth step the so obtained mixture is freed partially or completely from the solvent or mixture of solvents.

5. The process according to claim 4, **characterized in that** the carrier material is selected from the group, consisting of
- polyethylene glycols with an average molecular weight in the range from 300 to 600, especially 300,
- fatty acid esters of polyglycerines,
- lecithins,
- silicone oils,
- sorbitan fatty acid esters,
- sorbates of fatty acids,
- polysorbates of fatty acids,
- waxes,
- polyglycerines,
- triglycerides,
- fatty acids,
- fatty oils, and
- paraffins,
including any mixtures thereof.

6. The process according to one of claims 4 to 5, **characterized in that** to the at the end of the fourth step obtained mixture is added such an amount of water and/or glycerol and/or propylene glycol, that a corresponding content thereof from 5 % by weight to 20 % by weight, especially from 12 % by weight to 15 % by weight, results.

7. The process according to one of claims 1 to 6, **characterized in that** the solvent for the preparation of the plant extract is a polar solvent, especially selected from the group, consisting of
- water
- alcohols, especially a C₁ to C₄ alcohol, preferably ethanol, glycerol or propylene glycol,
- ketones, especially a C₃ to C₅ ketone, preferably aceton, and
- esters, especially an alkyl acetate, whereby the alkyl group has preferably 1 to 4 C-atoms,
including any mixtures thereof, for example a mixture of water and ethanol.

8. The process according to one of claims 1 to 7, **characterized in that** the agent A is a polymeric compound, and is especially selected from the group, consisting of
- polyvinylpyrrolidones, abbreviated with PVP,
- vinylacetat-crotonic acid-copolymers,
- methacrylic acid-ethylacrylate-copolymers,
- blockcopolymers from polyethylene glycols and polypropylene glycols,
- proteins and protein hydrolysates of proteins of plant and/or animal origin, for example gelatins, and
- blockcopolymers of ethylene oxide and propylene oxide,
including any mixtures thereof.

9. The process according to one of claims 1 to 8, **characterized in that**
- the weight ratio between the used plant and the used extraction agent is from 1:1 to 1:30, especially from 1:5 to 1:15, preferably from 1:4 to 1:8,
- the weight ratio between the used plant and the used agent A is in the case of
-- PVP from 5:1 to 120:1,
-- vinylacetate-crotonic acid-copolymers from 10:1 to 50:1,
-- methacrylic acid-ethylacrylate-copolymers from 10:1 to 50:1,
-- blockcopolymers of polyethylene glycols and polypropylene glycols from 2:1 to 50:1,
-- proteins and protein hydrolysates of proteins of plant and/or animal origin from 2:1 to 50:1,
-- blockcopolymers of ethylene oxide and propylene oxide from 2:1 to 50:1.

10. The process according to one of claims 1 to 9, **characterized in that** a plant or parts thereof of the family of the Papaveraceas, for example Chelidonium majus L., Fumaria officinalis L., Sanguinaria canadensis L., is mixed and extracted with a water/alcohol mixture, especially a mixture of water and ethanol, the so obtained extract is separated from the insoluble substances, the so obtained solution is mixed with a PVP, especially a PVP with an average molecular weight in the range from 1'000'000 to 1'500'000, whereby the weight ratio between the used plant and the used PVP is from 10:1 to 60:1, and occasionally the so obtained mixture is concentrated to a spissum extract having a water content from 55 % by weight to 75 % by weight, referred to the spissum extract, especially at a temperature of below 70°C and a pressure of less than 200 mbar, then is added to the so obtained mixture an agent, which increases the melting point, for example silicic acid, preferably precipitated silicic acid, and this mixture is dried.

11. The process according to one of claims 1 to 9, **characterized in that** a plant or parts thereof of the family of the Fabaceas, for example Melilotus officinalis L. Lam. Em. Thuill., Phaseolus vulgaris L., Astragalus-species, is mixed and extracted with a water/alcohol mixture, especially a mixture of water and ethanol, the so obtained extract is separated from the insoluble substances, the so obtained solution is mixed with a PVP, especially a PVP with an average molecular weight in the range from 2'000 to 54'000, especially 7'000 to 11'000, whereby the weight ratio between the used plant and the used PVP is from 10:1 to 40:1, and occasionally the so obtained mixture is concentrated to a spissum extract having a water content from 40 % by weight to 70 % by weight, referred to the spissum extract, especially at a temperature of below 50°C and a pressure of less than 80 mbar, preferably at a temperature from 30°C to 40°C and a pressure of less than 50 mbar, and this mixture is dried.

12. The process according to one of claims 1 to 9, **characterized in that** a plant or parts thereof of the family of the Liliaceas, for example Allium-species (e.g. A. cepa L., A. ursinum L., A. sativum L.: Bulbus), is mixed and extracted with a water/alcohol mixture, especially a mixture of water and ethanol, the so obtained extract is separated from the insoluble substances, the so obtained solution is mixed with a PVP, especially a PVP with an average molecular weight in the range from 2'000 to 54'000, especially 28'000 to 34'000, whereby the weight ratio between the used plant and the used PVP is from 20:1 to 120:1, especially 90:1, and occasionally the so obtained mixture is concentrated to a spissum extract having a water content from 30 % by weight to 60 % by weight, referred to the spissum extract, especially at a temperature of below 70°C and a pressure of less than 200 mbar, then is added to the so obtained mixture occasionally an antioxidant, for example α-tocopherol, and this mixture is dried.

13. Use of polymeric compounds, especially selected from the group, consisting of
- polyvinylpyrrolidones, abbreviated with PVP,
- vinylacetat - crotonic acid - copolymers,
- methacrylic acid-ethylacrylate-copolymers,
- blockcopolymers from polyethylene glycols and polypropylene glycols,
- proteins and protein hydrolysates of proteins of plant and/or animal origin, for example gelatins, and
- blockcopolymers of ethylene oxide and propylene oxide,
including any mixtures thereof,
as means for carrying out the process according to one of claims 1 to 12.

14. A stable, homogeneous, from secondary reaction products free or nearly free extract from plants, which are selected from the group as mentioned in claim 1, or parts thereof, **characterized in that** this extract contains beside the respective desired genuine substance mixture in a complete or nearly complete form also at least one agent A, whereby this agent A
- increases the solubility of at least one in the extract contained substance,
- increases the viscosity of the extract in the presence of a solvent or a mixture of solvents,
- influences the steam pressure of the in the extract occasionally contained volatile substances,
- prevents or at least reduces considerably the chemical and/or enzymatic reactions between one or more in the extract contained substance(s),
- prevents or at least reduces considerably through interaction with one or more in the extract contained substance(s) its(their) sedimentation and/or flotation, when the extract is in liquid, semisolid or in at least one solvent dissolved and/or suspended and/or emulsified form, and
- increases the stability and/or the homogeneity of at least one in the extract contained substance,
and
is in liquid, semisolid, solid or in at least one solvent dissolved and/or suspended and/or emulsified form.

15. The extract according to claim 14, **characterized in that** the solvents are pharmaceutically acceptable solvents, especially selected from the group, consisting of water, glycerol, propylenglycol, polyethylene glycols with an average molecular weight in the range from 300 to 1'500, especially 300, whereby a corresponding content thereof from 5 % by weight to 20 % by weight, especially from 12 % by weight to 15 % by weight, is preferred.

16. The extract according to one of claims 14 to 15, **characterized in that** it contains additionally at least one pharmaceutical acceptable additive and/or auxiliary agent, especially selected from the group, consisting of emulsifiers, stabilizers, antioxidants, dyestuffs, aromas, disintegration agents, agents, which have a favorable influence onto the free-flowing behavior, agents, which have a favorable influence onto the comprimation, agents, which increase the melting point, and agents, which reduce the hygroscopicity.

17. The extract according to one of claims 14 to 16, **characterized in that** it is mixed with at least one carrier material, whereby this carrier material must be encapsulatable and must be inert against all in the extract contained substances, and whereby the carrier material is preferably selected from the group, consisting of
- polyethylene glycols with an average molecular weight in the range from 300 to 600, especially 300,
- fatty acid esters of polyglycerines,
- lecithins,
- silicone oils,
- sorbitan fatty acid esters,
- sorbates of fatty acids,
- polysorbates of fatty acids,
- waxes,
- polyglycerines,
- triglycerides,
- fatty acids,
- fatty oils, and
- paraffins,
including any mixtures thereof.

18. Extract according to one of claims 14 to 17, **characterized in that** the agent A is a polymeric compound, and is especially selected from the group, consisting of
- polyvinylpyrrolidones, abbreviated with PVP,
- vinylacetat-crotonic acid-copolymers,
- methacrylic acid-ethylacrylate-copolymers,
- blockcopolymers from polyethylene glycols and polypropylene glycols,
- proteins and protein hydrolysates of proteins of plant and/or animal origin, for example gelatins, and
- blockcopolymers of ethylene oxide and propylene oxide,
including any mixtures thereof.

19. Extract according to one of claims 14 to 18, **characterized in that** the weight ratio between the genuine substance mixture, referred to the genuine dry substance, and the used agent A is in the case of
- PVP from 95:5 to 70:30,
- vinylacetate-crotonic acid-copolymers from 98:2 to 90:10,
- methacrylic acid-ethylacrylate-copolymers from 98:2 to 90:10,
- blockcopolymers of polyethylene glycols and polypropylene glycols from 98:2 to 90:10,
- proteins and protein hydrolysates of proteins of plant and/or animal origin from 98:2 to 70:30,
- blockcopolymers of ethylene oxide and propylene oxide from 98:2 to 90:10.

20. Extract according to one of claims 14 to 19, **characterized in that** the for the preparation of the genuine substance mixture used plants are selected from the group as mentioned in claim 1, whereby are preferred the families of the Papaveraceas, for example Chelidonium majus L., Fumaria officinalis L, Sanguinaria canadensis L., Fabaceas, for example Melilotus officinalis L. Lam. em. Thuill., Phaseolus vulgaris L., Astragalus-species, and Liliaceas, for example Allium-species, (e.g. A.cepa L., A. ursinum L., A. sativum L.: Bulbus).

21. Extract according to one of claims 14 to 20, **characterized in that** it is contained in a pharmaceutical acceptable administrative form, especially in
- solid administrative forms for oral application, especially in the form of a tablet, a film tablet, a dragee, a pellet, a hard gelatin-capsule, a soft gelatin-capsule,
- liquid administrative forms for oral, parenteral, rectal, vaginal and topic application, especially in the form of a dropping solution, a spray, an injection solution, a syrup,
- semisolid administrative forms for topic, oral, rectal and vaginal application, especially in the form of a cream, a gel, an ointment, a paste, a suppository.

22. Extract according to one of claims 14 to 21, **characterized in that** the plant used for the preparation of the genuine substance mixture is Chelidonium majus L., and that the agent A is a PVP with an average molecular weight in the range from 1'000'000 to 1'500'000.

23. Extract according to one of claims 14 to 21, **characterized in that** the plant used for the preparation of the genuine substance mixture is Allium sativum L., and that the agent A is a PVP with an average molecular weight in the range from 2'000 to 54'000, especially 28'000 to 34'000.

24. Extract according to one of claims 14 to 21, **characterized in that** the plant used for the preparation of the genuine substance mixture is selected from the group, consisting of Ammi visnaga L., Ephedra sinica (Stapf), Melilotus officinalis L. Lam. em. Thuill. and Zingiber officinale Roscoe, and that the agent A is a PVP with an average molecular weight in the range from 2'000 to 54'000, especially 7'000 to 11'000.

## Revendications

1. Procédé de préparation d'un extrait stable, homogène, exempt ou presque exempt de produits de réactions secondaires qui contient le mélange de substances d'origine respectivement souhaité sous forme complète ou quasi complète, cet extrait étant obtenu à partir de plantes ou de parties de celles-ci qui peuvent être utilisées à l'état frais et/ou séché, sous forme transformée ou non transformée, **caractérisé en ce que**
- dans une première étape
soit, selon une première variante
- A.) 1.) on mélange lesdites plantes ou parties de celles-ci dans un ordre quelconque avec au moins un solvant et au moins un agent, ci-après désigné agent A, on dissout et ensuite on extrait, étant entendu que cet agent A, par sa présence
- augmente la solubilité d'au moins une substance contenue dans l'extrait,
- augmente la viscosité de l'extrait en présence d'un solvant ou d'un mélange de solvants,
- influence la pression de vapeur des substances volatiles éventuellement contenues dans l'extrait,
- prévient ou, du moins, réduit considérablement les réactions chimiques et/ou enzymatiques entre une ou plusieurs substances contenues dans l'extrait,
- par interaction avec une ou plusieurs des substances contenues dans l'extrait, prévient ou, du moins, réduit considérablement leur sédimentation et/ou flottation lorsque l'extrait est présent sous forme liquide, semi-solide ou dissoute dans au moins un solvant et/ou suspendue et/ou émulsionnée, et
- augmente la stabilité et/ou l'homogénéité d'au moins une substance contenue dans l'extrait,
et
- A.) 2.) on sépare l'extrait ainsi obtenu des substances non solubles,
ou, selon une deuxième variante
- B.) 1.) on mélange lesdites plantes ou parties de plantes avec au moins un solvant et on extrait,
- B.) 2.) on sépare l'extrait ainsi obtenu des substances non solubles et
- B.) 3.) on mélange cet extrait avec au moins un des agents A précités, l'agent A étant dissous dans l'extrait et
- dans une deuxième étape
- on recueille l'extrait obtenu selon la première étape dans lequel l'agent A est contenu et resté, les plantes étant sélectionnées dans le groupe composé comme suit:
Espèces d'allium (p. ex., A. capa L., A. ursinum L., A. sativum L.: Bulbus)
Ammi visnaga L.
Arnica montana L.
Asparagus officinalis L.
Avena sativa L.
Espèces d'Astralagus
Berberis vulgaris L.
Brassica nigra L.Koch
Calendula officinalis L.
Capsicum frutescens L.
Carum carvi L.
Chelidonium majus L.
Chrysanthemum parthenium
Chrystanthemum vulgare Asch.
Cimicifuga racemosa L.
Centella asiatica L.
Cucurbita pepo L.
Espèces de Curcuma
Ephedra sinica (Stapf) et autres espèces
Euphrasia offic. L.
Foeniculum vulgare Miller
Fumaria officinalis L.
Glycine max. L.
Ilex paraguariensis St. Hil.
Jasminum grandiflorum L.
Laurus nobilis L.
Matricaria chamomilla L.
Melitotus officinalis L. Lam.em. Thuill.
Olea europea L.
Orthosiphon stamineus Benth.
Panax ginseng Meyer
Petroselinium crispum (Mill.) Nym.
Peumus boldus (Molina)
Phaseolus vulgaris L.
Pimenta dioica L. Merill
Pimpinella anisum L.
Plantago lanceolata L.
Espèces de Rauvolfia
Salix alba L. et toutes les espèces
Sanguinaria canadensis L.
Schisandra chinensis Baill.
Espèces de Smilax
Sylibum marianum L. Gaertner
Taraxacum officinale Web.
Urtica dioica L.
Valeriana officinalis L. et ses variétés
Vitex agnus castus L.
Zingiber officinale Roscoe

2. Procédé selon la revendication 1, **caractérisé en ce que** l'extrait obtenu à la fin de la deuxième étape est concentré en un extrait épais, en particulier à une température inférieure à 70°C, la pression respectivement utilisée étant déterminée selon le ou les solvants utilisés et, éventuellement,
- soit l'extrait épais ainsi obtenu est séché, de préférence à l'aide d'un dispositif à bande sous vide afin d'obtenir ainsi un extrait sec qui est à l'état aggloméré solide à une température inférieure à 60°C;
- soit l'extrait épais ainsi obtenu est mélangé à au moins un solvant acceptable pharmaceutiquement , en particulier sélectionné dans le groupe composé de l'eau, du glycérol, du propylèneglycol, de polyéthylèneglycols avec un poids moléculaire moyen de l'ordre de 300 à 1500, en particulier de 300 pour obtenir ainsi une préparation qui est à l'état aggloméré liquide à une température inférieure à 50°C.

3. Procédé selon l'une des revendications 1 à 2, **caractérisé en ce que** l'on ajoute encore au mélange à un moment quelconque, de préférence à la fin de la deuxième étape ou avant le séchage ou avant le mélange avec un solvant acceptable pharmaceutiquement au moins un additif et/ou adjuvant acceptable pharmaceutiquement, en particulier sélectionné dans le groupe composé des émulsifiants, des stabilisateurs, des antioxydants, des colorants, des arômes, des agents désintégrants, des agents qui influencent favorablement la faculté d'écoulement, des agents qui influencent favorablement le compactage, des agents qui augmentent le point de fusion et des agents qui réduisent l'hygroscopie.

4. Procédé selon la revendication 1, **caractérisé en ce**
- **que**, dans une troisième étape, l'extrait obtenu à la fin de la deuxième étape est mélangé avec au moins une substance excipiente pour ledit extrait, cette substance excipiente étant encapsulable et devant être inerte par rapport à toutes les substances présentes dans le mélange ainsi obtenu et
- **que**, dans une quatrième étape, le mélange ainsi obtenu est libéré en tout ou en partie du solvant ou du mélange de solvants.

5. Procédé selon la revendication 4, **caractérisé en ce que** la substance excipiente est sélectionnée dans le groupe composé:
- de polyéthylèneglycols avec un poids moléculaire moyen de l'ordre de 300 à 600, en particulier de 300,
- d'esters d'acide gras de polyglycérines,
- de lécithines,
- d'huiles de silicone,
- d'esters d'acides gras de sorbinate,
- de sorbates d'acides gras,
- de polysorbates d'acides gras,
- de cires,
- de polyglycérines,
- de triglycérides,
- d'acides gras,
- d'huiles grasses, et
- de paraffines,
y compris de quelconques mélanges de ces substances.

6. Procédé selon l'une des revendications 4 à 5, **caractérisé en ce qu'**on ajoute au mélange obtenu au terme de la quatrième étape assez d'eau et/ou de glycérol et/ou de propylèneglycol pour qu'un taux correspondant de ceux-ci de 5% en poids à 20% en poids, en particulier de 12% en poids à 15% en poids soit obtenu.

7. Procédé selon l'une des revendications 1 à 6, **caractérisé en ce que** le solvant pour la préparation de l'extrait de plantes est un solvant polaire, en particulier sélectionné dans le groupe composé
- de l'eau,
- d'alcools, en particulier d'un alcool C₁ à C₄, de préférence l'éthanol, le glycérol, ou le propylèneglycol,
- de cétones, en particulier d'une cétone C₃ à C₅, de préférence l'acétone, et
- d'esters, en particulier d'un acétate alkylique, le résidu alkyle ayant de préférence 1 à 4 atomes C,
y compris de quelconques mélanges de ceux-ci , par exemple ur mélange d'eau et d'éthanol.

8. Procédé selon l'une des revendications 1 à 7, **caractérisé en ce que** l'agent A est un composé polymère et est en particulier sélectionné dans le groupe composé
- de polyvinylpyrrolidones, en abrégé PVP,
- de copolymères d'acétate vinylique/acide crotonique,
- de copolymères d'acide méthacrylique-éthylacrylate,
- de copolymères blocs de polyéthylèneglycols et de polypropylèneglycols,
- de protéines et d'hydrolysats de protéine à partir de protéines d'origine animale et/ou végétale, par exemple des gélatines, et
- de copolymères blocs d'éthylèneoxyde et de propylèneoxyde,
y compris de quelconques mélanges de ceux-ci.

9. Procédé selon l'une des revendications 1 à 8, **caractérisé en ce**
- **que** le rapport pondéral de la plante utilisée à l'agent d'extraction mis en oeuvre est de 1:1 à 1:30, en particulier de 1:5 à 1:15, de préférence de 1:4 à 1:8,
- le rapport pondéral de la plante utilisée à l'agent A mis en oeuvre s'élève:
- à 5:1 à 120:1 en cas de PVP,
- à 10:1 à 50:1 en cas de copolymères d'acétate vinylique-acide crotonique,
- à 10:1 à 50:1 en cas de copolymères d'acide méthacrylique-acrylate éthylique,
- à 2:1 à 50:1 en cas de copolymères blocs de polyéthylèneglycols et de polypropylèneglycols,
- à 2:1 à 50:1 en cas de protéines et d'hydrolysats de protéines provenant de protéines d'origine végétale et/ou animale,
- à 2:1 à 50:1 en cas de copolymères blocs d'éthylèneoxyde et de propylèneoxyde.

10. Procédé selon l'une des revendications 1 à 9, **caractérisé en ce qu'**une plante ou des parties de celle-ci de la famille des papavéracées, par exemple le Chelidonium majus L., la Fumaria officinalis L., la Sanguinaria canadensis L. sont mélangées avec un mélange d'eau et d'alcool, en particulier un mélange d'eau et d'éthanol, puis extraites, l'extrait ainsi obtenu est séparé des substances insolubles, la solution ainsi obtenue est mélangée avec une PVP, en particulier une PVP d'un poids moléculaire moyen de l'ordre de 1.000.000 à 1.500.000, le rapport pondéral de la plante utilisée à la PVP mise en oeuvre allant de 10:1 à 60:1 et, éventuellement, le mélange ainsi obtenu est concentré en un extrait épais avec une teneur en eau de 55% en poids à 75% en poids par rapport à l'extrait épais, en particulier à une température inférieure à 70°C et une pression de moins de 200 mbars, un agent qui augmente le point de fusion est ajouté au mélange ainsi obtenu, par exemple l'acide silicique, de préférence l'acide silicique précipité et ce mélange est séché.

11. Procédé selon l'une des revendications 1 à 9, **caractérisé en ce qu'**une plante ou des parties de celle-ci de la famille des fabacées, par exemple le Melitotus officinalis L. Lam. Em. Thuill., le phaseolus vulgaris L., les espèces d'Astragalus, sont mélangées avec un mélange d'eau et d'alcool, en particulier un mélange d'eau et d'éthanol, puis extraites, l'extrait ainsi obtenu est séparé des substances insolubles, la solution ainsi obtenue est mélangée avec une PVP, en particulier une PVP avec un poids moléculaire moyen de l'ordre de 2.000 à 54.000, en particulier 7.000 à 11.000, le rapport pondéral de la plante utilisée à la PVP mise en oeuvre allant de 10:1 à 40:1 et, éventuellement, le mélange ainsi obtenu est concentré en un extrait épais avec une teneur en eau de 40% en poids à 70% en poids par rapport à l'extrait épais, en particulier à une température inférieure à 50°C et une pression de moins de 80 mbars, de préférence à une température de 30°C à 40°C et à une pression de moins de 50 mbars, et ce mélange est séché.

12. Procédé selon l'une des revendications 1 à 9, **caractérisé en ce qu'**une plante ou des parties de celle-ci de la famille des liliacées, par exemple les espèces d'Allium (p. ex. E. cepa L.; A. ursinum L., A. sativum L.: Bulbus) sont mélangées avec un mélange d'eau et d'alcool, en particulier un mélange d'eau et d'éthanol, puis extraites, l'extrait ainsi obtenu est séparé des substances insolubles, la solution ainsi obtenue est mélangée avec une PVP, en particulier une PVP avec un poids moléculaire moyen de l'ordre de 2.000 à 54.000, en particulier 28.000 à 34.000, le rapport pondéral de la plante utilisée à la PVP mise en oeuvre allant de 20:1 à 120:1, en particulier 90:1, et, éventuellement, le mélange ainsi obtenu est concentré en un extrait épais avec une teneur en eau de 30% en poids à 60% en poids par rapport à l'extrait épais, en particulier à une température inférieure à 70°C et une pression de moins de 200 mbars, un antioxydant est éventuellement ajouté au mélange ainsi obtenu, par exemple l'α-tocophérol, et ce mélange est séché.

13. Utilisation de composés polymères, en particulier sélectionnés dans le groupe composé
- de polyvinylpyrrolidones, en abrégé PVP,
- de copolymères d'acétate vinylique/acide crotonique,
- de copolymères d'acide méthacrylique-éthylacrylate,
- de copolymères blocs de polyéthylèneglycols et de polypropylèneglycols,
- de protéines et d'hydrolysats de protéine à partir de protéines d'origine animale et/ou végétale, par exemple des gélatines, et
- de copolymères blocs d'éthylèneoxyde et de propylèneoxyde,
y compris de quelconques mélanges de ceux-ci,
comme agent pour la mise en oeuvre du procédé selon l'une des revendications 1 à 12.

14. Extrait stable, homogène, exempt ou presque exempt de produits de réactions secondaires à partir de plantes qui sont sélectionnées dans le groupe spécifié à la revendication 1 ou de parties de celles-ci, **caractérisé en ce que** cet extrait contient, outre le mélange de substances d'origine respectivement souhaité sous forme complète, ou quasi complète, encore au moins un agent A, auquel cas cet agent A
- augmente la solubilité d'au moins une substance contenue dans l'extrait,
- augmente la viscosité de l'extrait en présence d'un solvant ou d'un mélange de solvants,
- influence la pression de vapeur des substances volatiles éventuellement contenues dans l'extrait,
- prévient ou, du moins, réduit considérablement les réactions chimiques et/ou enzymatiques entre une ou plusieurs substances contenues dans l'extrait,
- par interaction avec une ou plusieurs des substances contenues dans l'extrait, prévient ou, du moins, réduit considérablement leur sédimentation et/ou flottation lorsque l'extrait est présent sous forme liquide, semi-solide ou dissoute dans au moins un solvant et/ou suspendue et/ou émulsionnée, et
- augmente la stabilité et/ou l'homogénéité d'au moins une substance contenue dans l'extrait,
et
est présent sous forme liquide, semi-solide, solide ou dissoute dans au moins un solvant et/ou mis en suspension et/ou émulsionné.

15. Extrait selon la revendication 14, **caractérisé en ce que** les solvants sont des solvants pharmaceutiquement acceptables, en particulier sélectionnés dans le groupe composé de l'eau, du glycérol, du propylènegycol, de polyéthylèneglycols d'un poids moléculaire moyen de l'ordre de 300 à 1500, en particulier de 300, un taux correspondant de 5% en poids à 20% en poids, en particulier de 12% en poids à 15% en poids étant préféré.

16. Extrait selon l'une des revendications 14 à 15, **caractérisé en ce qu'**il contient encore au moins un additif et/ou adjuvant acceptable pharmaceutiquement, en particulier sélectionné dans le groupe composé des émulsifiants, des stabilisateurs, des antioxydants, des colorants, des arômes, des agents désintégrants, des agents qui influencent favorablement la faculté d'écoulement, des agents qui influencent favorablement le compactage, des agents qui augmentent le point de fusion et des agents qui réduisent l'hygroscopie.

17. Extrait selon l'une des revendications 14 à 16, **caractérisé en ce qu'**il est mélangé avec au moins une substance excipiente, cette substance excipiente étant encapsulable et devant être inerte par rapport à toutes les substances présentes dans l'extrait et la substance excipiente est sélectionnée de préférence dans le groupe composé:
- de polyéthylèneglycols avec un poids moléculaire moyen de l'ordre de 300 à 600, en particulier de 300,
- d'esters d'acide gras de polyglycérines,
- de lécithines,
- d'huiles de silicone,
- d'esters d'acides gras de sorbinate,
- de sorbates d'acides gras,
- de polysorbates d'acides gras,
- de cires,
- de polyglycérines,
- de triglycérides,
- d'acides gras,
- d'huiles grasses, et
- de paraffines,
y compris de quelconques mélanges de ces substances.

18. Extrait selon l'une des revendications 14 à 17, **caractérisé en ce que** l'agent A est un composé polymère et est en partie sélectionné dans le groupe composé
- de polyvinylpyrrolidones, en abrégé PVP,
- de copolymères d'acétate vinylique/acide crotonique,
- de copolymères d'acide méthacrylique-éthylacrylate,
- de copolymères blocs de polyéthylèneglycols et de polypropylèneglycols,
- de protéines et d'hydrolysats de protéine à partir de protéines d'origine animale et/ou végétale, par exemple des gélatines, et
- de copolymères blocs d'éthylèneoxyde et de propylèneoxyde,
y compris de quelconques mélanges de ceux-ci.

19. Extrait selon l'une des revendications 14 à 18, **caractérisé en ce que** le rapport pondéral du mélange de substances d'origine, par rapport à la substance sèche d'origine, à l'agent A utilisé s'élève à:
- 95:5 à 70:30 dans le cas de la PVP,
- 98:2 à 90:10 en cas de copolymères d'acétate vinylique/acide crotonique,
- 98:2 à 90:10 en cas de copolymères d'acide méthacrylique-éthylacrylate,
- 98:2 à 90:10 en cas de copolymères blocs de polyéthylèneglycols et de polypropylèneglycols,
- 98:2 à 70:30 en cas de protéines et d'hydrolysats de protéine à partir de protéines d'origine animale et/ou végétale,
- 98:2 à 90:10 en cas de polymères blocs d'éthylèneoxyde et de propylèneoxyde.

20. Extrait selon l'une des revendications 14 à 19, **caractérisé en ce que** les plantes utilisées pour la préparation du mélange de substances d'origine sont sélectionnées dans le groupe spécifié à la revendication 1, les familles des papavéracées, par exemple le Chelidonium majus L., la Fumaria officinalis L., la Sanguinaria canadensis L., les fabacées, par exemple le Melitotus officinalis L. Lam. em. Thuill., Phaseolus vulgaris L., les espèces d'Astragalus et les liliacées, par exemple les espèces d'Allium (p.ex., A. cepa L., A. ursinum L., A. sativum L.: Bulbus) étant préférées.

21. Extrait selon l'une des revendications 14 à 20, **caractérisé en ce qu'**il est contenu dans une forme d'administration acceptable pharmaceutiquement, en particulier
- dans des formes d'administration solides en vue d'une application orale, en particulier sous la forme d'un comprimé, d'un comprimé enrobé, d'une dragée, d'un granulé, d'une gélule en gélatine dure, d'une gélule en gélatine souple;
- dans des formes d'administration liquides pour une application orale, parentérale, rectale, vaginale et locale, en particulier sous la forme d'une solution en gouttes, d'un spray, d'une solution d'injection, d'un sirop,
- dans des formes d'administration semi-solides pour une application locale, orale, rectale et vaginale, en particulier sous la forme d'une crème, d'un gel, d'une pommade, d'une pâte, d'un suppositoire.

22. Extrait selon l'une des revendications 14 à 21, **caractérisé en ce que** la plante utilisée pour la préparation du mélange de substances d'origine est le Chelidonium majus L. et que l'agent A est une PVP d'un poids moléculaire moyen de l'ordre de 1.000.000 à 1.500.000.

23. Extrait selon l'une des revendications 14 à 21, **caractérisé en ce que** la plante utilisée pour la préparation du mélange de substances d'origine est l'Allium sativum L. et que l'agent A est une PVP d'un poids moléculaire moyen de l'ordre de 2.000 à 54.000, en particulier 28.000 à 34.000.

24. Extrait selon l'une des revendications 14 à 21, **caractérisé en ce que** la plante utilisée pour la préparation du mélange de substances d'origine est choisie dans le groupe composé de l'Ammi visnaga L., de l'Ephedra sinica (Stapf), du Melilotus officinalis L. Lam. em. Thuill. et du Zingiber officinale Roscoe et que l'agent A est une PVP d'un poids moléculaire moyen de l'ordre de 2.000 à 54.000, en particulier 7.000 à 11.000.
